# EUROPEAN PATENT SPECIFICATION

(11) **EP 1 292 299 B1**
(45) Date of publication and mention of the grant of the patent: **07.04.2004**
(21) Application number: 01929832.2
(22) Date of filing: 17.05.2001
(51) Int. Cl.: A61K 31/42, A61K 31/352, A61K 31/122, A61K 35/74, A61P 35/00

(54) **NEW USE OF CITREAMICINS**
NEUE VERWENDUNG VON CITREAMICINEN
NOUVELLE UTILISATION DE CITREAMICINES

(30) Priority: 17.05.2000 GB 0011927
(43) Date of publication of application: 19.03.2003
(73) Proprietor: Instituto Biomar S.A., 28760 Madrid (ES)
(72) Inventor: FERNANDEZ PUENTES, José Luis Instituto Biomar S.A., E-24231 Onzonilla Leon (ES); CANEDO FERNANDEZ, Librada, M Instituto Biomar S.A., E-24231 Onzonilla Leon (ES); GARCIA GRAVALOS, Dolores Pharma Mar, S.A., E-27860 Tres Cantos (ES); PEREZ-BAZ, Julia Instituto Biomar S.A., E-24231 Onzonilla Leon (ES); ROMERO-MILLAN, Francisco Instituto Biomar S.A., E-24231 Onzonilla Leon (ES); ESPLIEGO-VAZQUEZ, Fernando Instituto Biomar S.A., E-24231 Onzonilla Leon (ES)
(74) Representative: Ruffles, Graham Keith
(86) International application number: PCT/GB2001/002148
(87) International publication number: WO 2001/087283

(56) References cited:
- EP-A- 0 353 381
- EP-A- 0 405 151
- EP-A- 0 442 003
- PEARCE, C. J. ET AL: "The effect of methylation inhibitors on citreamicin biosynthesis in Micromonospora citrea" THE JOURNAL OF ANTIBIOTICS, vol. 44, no. 10, November 1991 (1991-11), pages 1247-1251, XP001038164
- QADRI, S. M. HUSSAIN ET AL: "Antibacterical activity of citramicin-alpha (LL-E 19085 alpha) against Gram-positive cocci" CHEMOTHERAPY, vol. 38, 1992, pages 395-398, XP001038166
- CARTER, T. GUY ET AL: "Citramicins, novel antibiotics from Micromonospora citrea: isolation, characterization, and structure determination" THE JOURNAL OF ANTIBIOTICS, vol. 43, no. 5, May 1990 (1990-05), pages 504-512, XP001038172
- MAIESE, W.M. ET AL.: "LL-E19085 alpha, a novel antibiotic from Micromonospora citrea: taxonomy, fermentation and biological activity" THE JOURNAL OF ANTIBIOTICS, vol. 42, no. 6, June 1989 (1989-06), page 846-51 XP002188244

## Description

The present invention relates to citreamicins, and in particular to a new use of the citreamicins.

### BACKGROUND

The citreamicins are known comopunds, see: Pearce, C.J.; Carter, G.T.; Nietsche, J.A.; Borders, D.B.; Greenstein, M.and Maiese, W.M. *J. Antibiotics,* **1991,** *44(11)*, 1247-1250. The citreamicins thus include compounds of the formula: 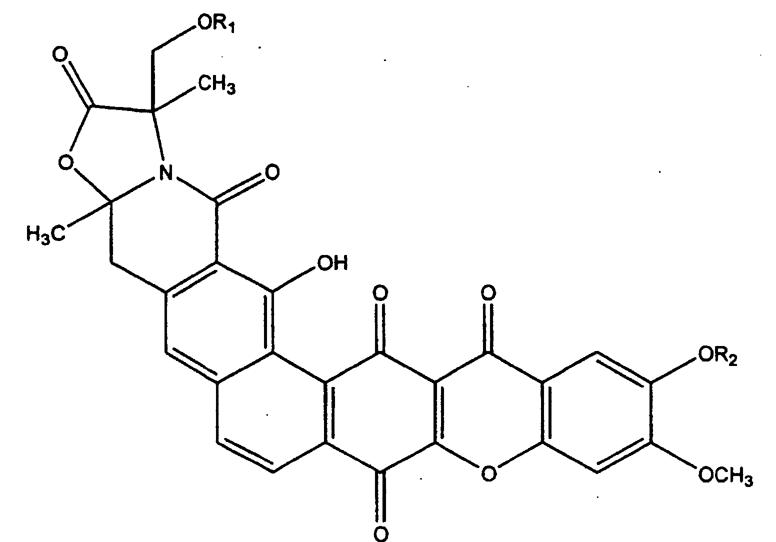 wherein R₁ is selected from the group consisting of COCH₂CH(CH₃)₂, COCH(CH₃)₂, COCH₃ or H when R₂ is CH₃, or wherein R₁ is COCH₂CH(CH₃)₂ and R₂ is H. In particular, citreamicin α is a compound of formula (I) where R₁ is COCH₂CH(CH₃)₂ and R₂ is CH₃.

### SUMMARY OF INVENTION

We have now found a new use of the known citreamicins, especially those of the formula (I). We have found that they exhibit antitumor activity.

Thus, we envisage the use of pharmaceutical compositions for treatment of tumors and which include a citreamicin and a pharmaceutically acceptable carrier.

We provide methods of making such pharmaceutical compositions, including the use of a citreamicin in the preparation of a medicament for use in treating a tumor.

The compositions are suited to be used in the manufacture of a medicament for treating a mammal affected by a malignant tumor sensitive to a citreamicin compound such as a compound of formula (I), which comprises administering to the affected individual a therapeutically effective amount of the citreamicin compound or a pharmaceutical composition thereof

### DETAILS OF THE INVENTION

Examples of pharmaceutical compositions include any solid (tablets, pills, capsules, granules, etc.) or liquid (solutions, suspensions or emulsions) with suitable formulation of oral, topical or parenteral administration, and they may contain the pure compound or in combination with any carrier or other pharmacologically active compounds. These compositions may need to be sterile when administered parenterally.

The correct dosage of a pharmaceutical composition comprising a citreamaicin compound will vary according to the pharmaceutical formulation, the mode of application, and the particular situs, host and tumor being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

We have found in particular that citreamicin α exhibits *in vitro* antitumor activity against a cell line derived from mouse lymphoma.

### BIOLOGICAL ACTIVITY

Citreamicin α displays good antitumor activity. Its antitumor activity has been detected *in vitro* by culturing the tumor cells following the methodology described by
(1): Raymond I. Bergeron, Paul F. Cavanaugh, Jr., Steven J. Mine, Robert G. Hughes, Jr., Gary T. Elliot and Carl W. Porter. Antineoplastic and antiherpetic activity of spermidine catecholamide iron chelators. *Biochem. Bioph. Res. Comin.* 1984, *121(3): 848-854*; and
(2). Alan C. Schroeder, Robert G. Hughes, Jr. and Alexander Bloch. Effects of Acycic Pyrimidine Nucleoside Analoges. *J. Med. Chem.* **1981,** 24:1078-1083.

Cells were maintained in logarithmic phase of growth in Eagle's Minimum Essential Medium, with Earle's Balanced Salts, with 2.0 mM L-glutamine, with non-essential amino acids, without sodium bicarbonate (EMEM/neaa); supplemented with 10% Fetal Calf Serum (FCS), 10⁻² M sodium bicarbonate and 0.1 g/l penicillin-G + streptomycin sulfate.

A screening procedure has been carried out to determine and compare the antitumor activity of citreamicin CL, using an adapted form of the method described by Bergeron et al. The tumor cells employed were P388 (ATCC CCL-46, suspension culture of a lymphoid neoplasm from DBA/2 mouse), A549 (ATCC CCL-185, monolayer culture of a human lung carcinoma) and HT-29 (ATCC HTB-38, monolayer culture of a human colon carcinoma).

P388 cells were seeded into 16 mm wells at 1 x 10⁴ cells per well in 1 ml aliquots of Minimum Essential Medium 5% Fetal Calf Serum, MEM 5FCS, containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, an approximate IC₅₀ was determined by comparing the growth in wells with drug to the growth in wells control.

A549 and HT-29 cells were seeded into 16 mm wells at 2 x 10⁴ cells per well in 1 ml aliquots of Minimum Essential Medium 10% Fetal Calf Serum, MEM 1OFCS, containing the indicated concentration of drug. A separate set of cultures without drug was seeded as control growth to ensure that cells remained in exponential phase of growth. All determinations were carried out in duplicate. After three days of incubation at 37°C, 10% CO₂ in a 98% humid atmosphere, the wells were stained with 0.1% Crystal Violet. An approximate IC₅₀ was determined by comparing the growth in wells with drug to the growth in wells control.

The activity results, IC₅₀(µM), for citreamicin α are given in the following table:

| | P388 | A549 | HT-29 |
|---|---|---|---|
| | ATCC CCL-46 | ATCC CCL-185 | ATCC HTB-38 |
| Citreamicin α | 0.003 | 0.004 | 0.004 |

## Claims

1. The use of a citreamicin in the preparation of a medicament for the treatment of a tumor.

2. The use according to claim 1, wherein the citreamicin is of the formula (I), 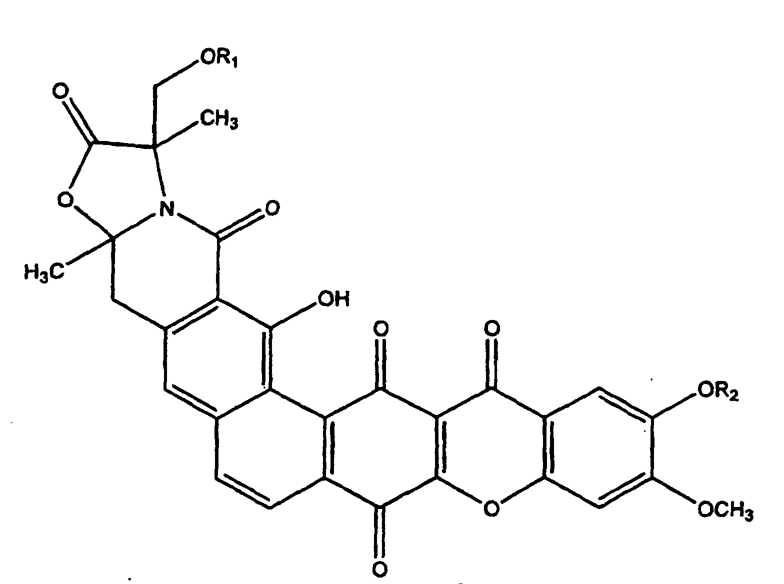 wherein R₁ is selected from the group consisting of COCH₂CH(CH₃)₂, COCH(CH₃)₂, COCH₃ or H when R₂ is CH₃, or wherein R₁ is COCH₂CH(CH₃)₂ and R₂ is H.

3. The use according to claim 2, wherein the citreamicin is citreamicin α which is of formula (I) where R₁ is COCH₂CH(CH₃)₂ and R₂ is CH₃.

## Patentansprüche

1. Verwendung eines Citreamicins bei der Herstellung eines Medikaments für die Behandlung eines Tumors.

2. Verwendung nach Anspruch 1, wobei das Citreamicin die Formel (I) hat, 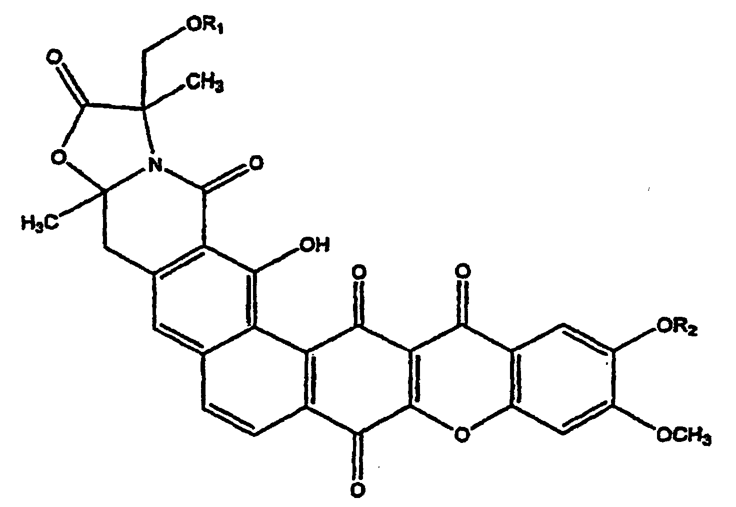 wobei R₁ aus der Gruppe, bestehend aus COCH₂CH(CH₃)₂, COCH(CH₃)₂, COCH₃ oder H, ausgewählt ist, wenn R₂ CH₃ ist, oder wobei R₁ COCH₂CH(CH₃)₂ ist und R₂ H ist.

3. Verwendung nach Anspruch 2, wobei das Citreamicin Citreamicin a ist, welches die Formel (I) hat, wo R₁ COCH₂CH(CH₃)₂ ist und R₂ CH₃ ist.

## Revendications

1. Utilisation d'une citréamicine dans la préparation d'un médicament pour le traitement d'une tumeur.

2. Utilisation selon la revendication 1, dans laquelle la citréamicine a la formule (I), 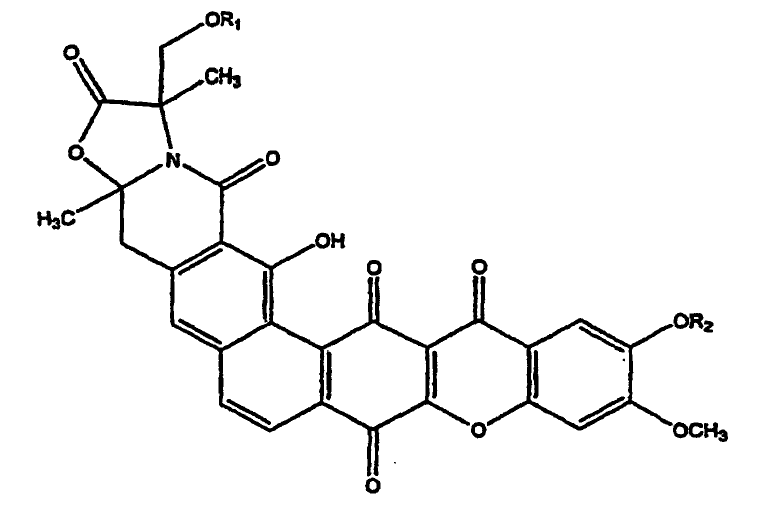 dans laquelle R₁ est choisi dans le groupe constitué par COCH₂CH(CH₃)₂, COCH(CH₃)₂, COCH₃ ou H lorsque R₂ est CH₃, ou dans laquelle R₁ est COCH₂CH(CH₃)₂ et R₂ est H.

3. Utilisation selon la revendication 2, dans laquelle la citréamicine est une citréamicine α qui a la formule (1) dans laquelle R₁ est COCH₂CH(CH₃)₂ et R₂ est CH₃.
